# EUROPEAN PATENT APPLICATION

(11) **EP 0 745 367 A2**
(43) Date of publication of application: **04.12.1996**
(21) Application number: 96201435.3
(22) Date of filing: 22.05.1996
(51) Int. Cl.: A61F 13/15

(54) **Disposable absorbent article**

(30) Priority: 30.05.1995 JP 132252/95
(71) Applicant: UNI-CHARM CORPORATION, Kawanoe-shi Ehime-ken (JP)
(72) Inventor: Kenmochi, Yasuhiko, Kanonji-shi, Kagawa-ken (JP); Mizutani, Satoshi, Kawanoe-shi, Ehime-ken (JP); Takai, Hisashi, Kawanoe-shi, Ehime-ken (JP)
(74) Representative: Van Malderen, Joelle

(57) **Abstract**

A disposable absorbent article being provided on its laterally opposite sides with a pair of barrier cuffs made of a plastic film. Each barrier cuff has a pair of walls opposed to each other with a space defined therebetween and a plurality of tubular passages extending from an outer surface of the wall to an inner surface thereof. Inlet and outlet openings of the passages are placed outside and inside of the wall, respectively.

## Description

The present invention relates generally to disposable body fluids absorbent articles and, more particularly, disposable body fluids absorbent articles such as disposable diapers, incontinent pads, sanitary napkins and the like for absorption and containment of urine, feces and menstrual discharge.
Conventional disposable absorbent articles comprising a laminate structure having a liquid-permeable topsheet, a liquid-impermeable backsheet and a liquid-absorbent core disposed between these two sheets , and a pair of body fluids barrier cuffs longitudinally extending on opposite sides of the laminate structure and normally biased to rise on the upper surface. Such cuffs are generally made of a nonwoven fabric of thermoplastic synthetic fibers and, preferably of a nonwoven fabric subjected to water-repellent finish so that the nonwoven fabric may obtain a breathability, and liquid permeation retarding property or liquid-impermeability. However, the known barrier cuffs made of a nonwoven fabric have been disadvantageous in that a quantity of body fluids may sometimes exude through fiber interstices. Particularly when the barrier cuff treated with a water repellent agent is in contact with body fluids for a long time, the water repellent agent commences to be dissolved in body fluids and facilitates such exudation of body fluids. It will be obviously possible to replace the nonwoven fabric by a plastic film in order to avoid such exudation. However, this can be achieved only at the cost of the breathability desired for the barrier cuffs and consequently the undergarment should inevitably become stuffy.

A principal feature of the invention is the provision of a disposable absorbent article capable of solving the above-mentioned problem left behind by the prior art by forming barrier cuffs of a plastic film having a plurality of tubular passages extending therethrough assuring a desired breathability.

Specifically, according to the invention, there is provided a disposable absorbent article comprising a laminate structure composed of a liquid-permeable topsheet, a liquid-impermeable backsheet and a liquid-absorbent core disposed between these two sheets, and a pair of barrier cuffs made of a plastic film and provided on transversely opposite sides of the laminate structure, wherein each the barrier cuffs is configured to have a pair of mutually opposed walls with a space defined therebetween and a plurality of tubular passages extending from their inlet openings defined on an outer surface of the wall to their outlet openings defined on an inner surface of the wall.

To implement the invention as defined above, a single film having the tubular passages may be folded in two or a pair of such films each having the tubular passages may be placed one upon another in order to form the barrier cuff. If desired, a liquid-absorbent material may be disposed within the space defined between the mutually opposite walls of the barrier cuff. Furthermore, the outlet openings of the respective tubular passages defined on one of the mutually opposite walls may be out of alignment with the outlet openings of the respective tubular passages defined on the other wall.

With the article constructed as described above, the presence of the tubular passages in the barrier cuff offers a good breathability between in- and exterior of the undergarment. A quantity of body fluids can move into the cuff through the tubular passages, but the quantity of body fluids once having moved into the cuff can not easily exude because the tubular passages distributed on the mutually opposite inner surface sections have their outlet openings mutually opposed but in relationship of discontinuity.

The embodiment of the invention in which the inner diameter of each tubular passage is tapered toward its outlet opening advantageously facilitates a quantity of body fluids to move into the barrier cuff under the capillary action occurring between body fluids and inner walls of the respective tubular passages. The liquid-absorbent material optionally disposed within the cuff positively absorbs a quantity of body fluids and contains it therein. The quantity of body fluids thus contained by the liquid-absorbent material can not easily back flow and leak through the tubular passages even when the barrier cuff is compressed.
Fig. 1 is a perspective view showing an embodiment of disposable diaper according to the invention as partially broken away;
Fig. 2 is a fragmentary perspective view of the diaper showing a section taken along a line II-II in Fig. 1;
Fig. 3 is a perspective view showing an embodiment of a barrier cuff;
Fig. 4 is a view similar to Fig. 3 showing an alternative embodiment of the barrier cuff; and
Fig. 5 is a view similar to Fig. 2 showing an alternative embodiment of the disposable diaper.

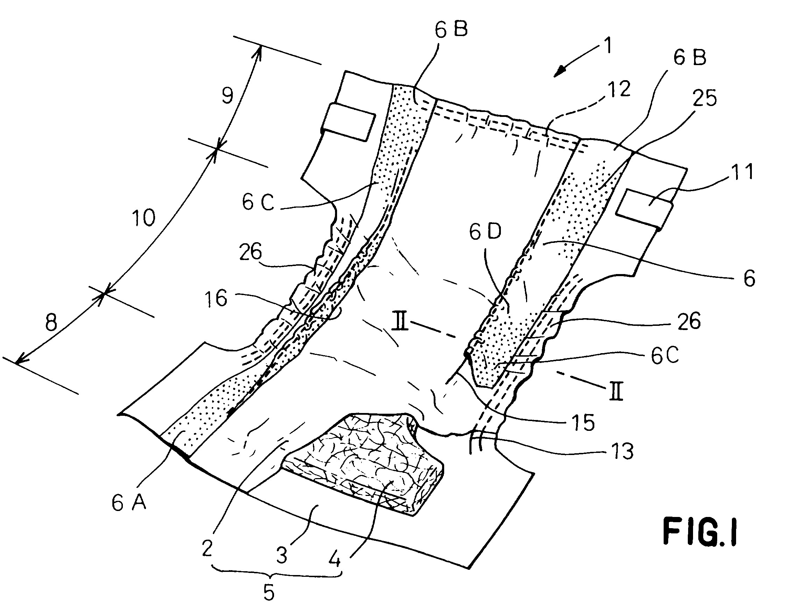

Referring to Fig. 1, a diaper 1 of the invention is illustrated in a perspective view as partially broken away. The diaper 1 comprises a diaper's laminate structure 5 composed of a liquid-permeable topsheet 2, a liquid-impermeable backsheet 3 and a liquid-absorbent core 4 disposed between these two sheets 2, 3, and a pair of barrier cuffs 6 longitudinally extending on an upper surface of the laminate structure 5 at either side thereof. In the laminate structure 5, the top- and backsheets 2, 3 are identical to each other in shape as well as in size and they are watertightly bonded to each other at their portions extending outward beyond a peripheral edge of the core 4. As viewed longitudinally, the laminate structure 5 is composed of a front section 8, a rear section 9 and a crotch section 10, the crotch section having its transversely opposite side edges cut out in arcs, respectively. The rear section 9 is provided with a pair of tape fasteners 11 attached to its transversely opposite sides. The rear section 9 is further provided along one of its longitudinally opposite end edges with an elastic member 12 for a waist-opening and the crotch section 10 is provided along its transversely opposite side edges with a pair of elastic members 13 for respective leg-openings, wherein the respective elastic members 12, 13 are bonded in an elastically contractible condition to the inner surface of at least any one of the top- and backsheets 2, 3. Each barrier cuff 6 is made of a plastic film 25 and bonded to the topsheet 2 along its proximal edge 6C and at their longitudinally opposite ends 6A, 6B. Each barrier cuff 6 is provided along its distal edge 6D with an elastic member 15 bonded thereto in an elastically contractible condition. The transversely opposite side edges of the crotch zone 10 along which the top- and backsheets 2, 3 are bonded to each other form a pair of leg-opening flaps 26 adapted to fit around the legs of a wearer under the contraction of the associated elastic members 13.

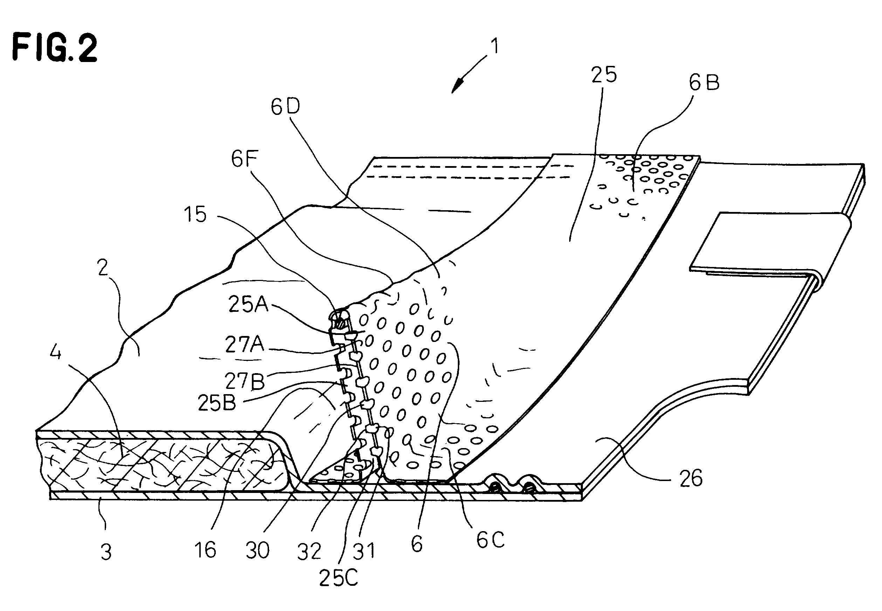

Referring to Fig. 2, the diaper 1 is illustrated partially in a perspective view and partially in a sectional view taken along a line II-II in Fig. 1. The proximal edge 6C of each barrier cuff 6 is bonded to the upper surface of the associated leg-opening flap 26 by any suitable means such as hot melt adhesive or heating. The elastic member 15 of each barrier cuff is expanded to collapse the barrier cuff 6 on an upper surface of the diaper 1 as the diaper 1 is longitudinally extended while the elastic member 15 is contracted to rise the barrier cuff 6 as the diaper 1 is curved as seen in Fig. 1. The barrier cuff 6 risen in this manner cooperates with the topsheet 2 to form pockets 16 opening inwardly of the diaper 1. The barrier cuff 6 comprises a plastic film 25 folded along a crest 6F in two to form a pair of mutually opposed walls 25A, 25B with a space 25C defined therebetween and the elastic member 15 bonded thereto inside the crest 6F. The barrier cuff 6 is provided with a plurality of tubular passages 30 extending through its thickness from its outer surface 27A to its inner surface 27B and projecting from its inner surface 27B, defining inlet and outlet openings 31, 32 in the outer and inner surfaces 26, 27B, respectively. Thus the barrier cuff 6 is formed by folding the film 25 with the outlet openings 32 of the tubular passages 30 being defined inside the barrier cuff 6, wherein the outlet openings 32 distributed on opposite inner surface sections of the barrier cuff 6 may be substantially in mutual alignment or out of such mutual alignment. The film 25 is preferably handled so that these outlet openings 32 may be out of mutual alignment. It should be understood that the proximal edge 6C of each barrier cuff 6 is bonded to the topsheet 2 with the tubular passages 30 being in their crushed condition. The longitudinally opposite ends 6A, 6B of the barrier cuff 6 are collapsed inwardly of the diaper 1 and bonded to the topsheet 2.

The outer and inner surfaces 26, 27, particularly the latter, of the film 25 are preferably treated with a hydrophilic agent for well known in the art for improving the movement of the body fluids from the outer surface 26 to the inner surface 27 though the passages 30.

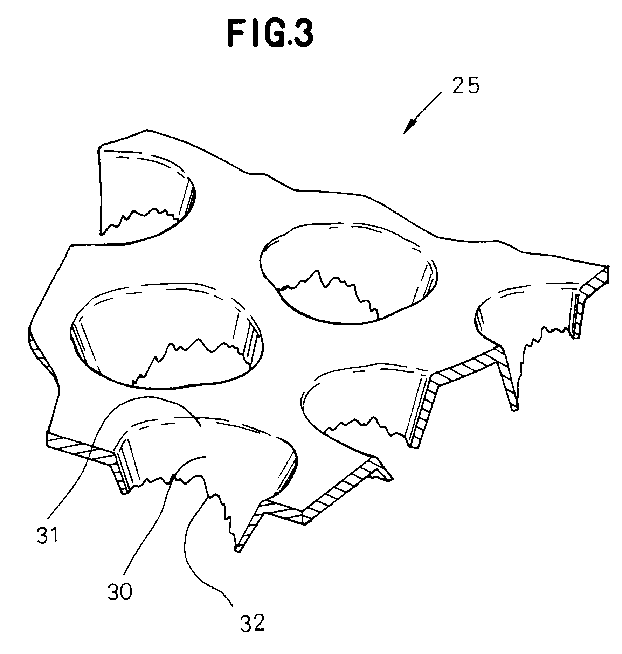

Fig. 3 is a scale-enlarged fragmentary perspective view exemplarily illustrating the perforated plastic film 25 disclosed in Japanese Laid-Open Patent Application No. Hei5 - 49659 filed by the applicant of the present application and being useful as a material for the barrier cuff 6. Such film 25 has the tubular passages 30 extending through its thickness from the outer surface to the inner surface and each of these tubular passages 30 has the inlet opening 31 and the outlet opening 32 distributed on the outer and inner surfaces, respectively. Accordingly, the film 25 may be folded to form the barrier cuff 6 with the outlet openings 32 being defined inside the barrier cuff 6. It should be understood that there may occur three cases, i.e., the case in which the tubular passage 30 progressively becomes smaller in its diameter, the case in which the tubular passage 30 progressively becomes larger in its diameter and the case in which the tubular passage 30 is substantially uniform in its diameter. In implementation of the invention, it is preferred to be adopted the tubular passage 30 which progressively becomes smaller in its diameter, i.e., which is tapered toward its outlet opening 32, since such tubular passage 30 facilitates body fluids discharged on the outer surface of the film 25 to move from the inlet opening 31 toward the outlet opening 32 under the capillary action occurring along the inner wall of the tubular passage 30 and restrains a significant back flow of the body fluids. In other words, the body fluids can easily move from the outer side to the inner side of the barrier cuff 6 but it is difficult for the body fluids to move from the inner side back to the outer side of the barrier cuff 6.

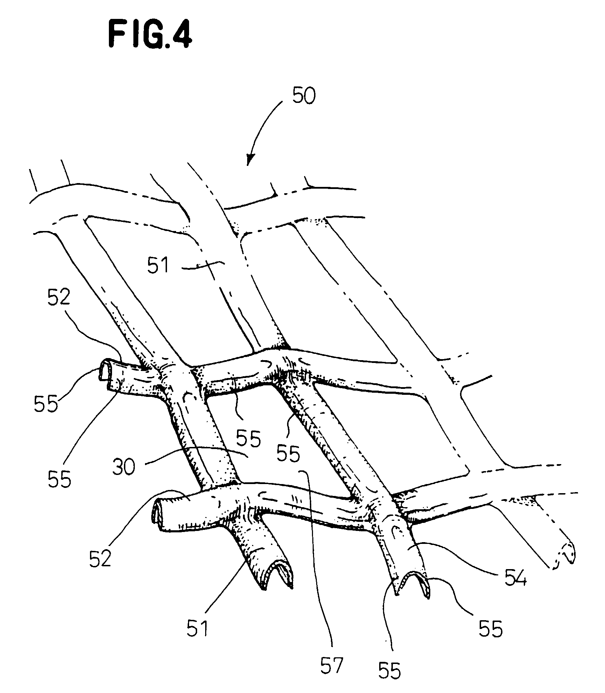

Fig. 4 is a scale-enlarged fragmentary perspective view exemplarily illustrating a plastic film 50 disclosed in Japanese Laid-Open Patent Application No. Hei4 - 82977 filed by the applicant of the present application and being useful as a material for the barrier cuff 6. Such film 50 is composed of a plurality of ribs 51 extending longitudinally of the film 50 and a plurality of ribs 52 transversely intersecting the ribs 51, wherein the adjacent pair of the ribs 51 intersect the adjacent pair of the ribs 52 so as to define an opening 57. Each rib 51, 52 presents a cross-section describing an arc which opens downward, i.e., curves down from an apex 54 so as to define side wall 55 on either side. In each opening 57, the side walls 55 of each rib 51, 52 are connected one to another completely to form the tubular passage 30. Such film 50 may be folded in two with the apices or ridgelines 54 of each rib 51, 52 outside and lower edges of the side walls 55 inside so as to form the barrier cuff 6. Such film 50 may be made of a plastic material such as polyethylene. Each rib 51, 52 preferably has a width of 0.1 to 2.0 mm and a height of 0.3 to 2.0 mm.

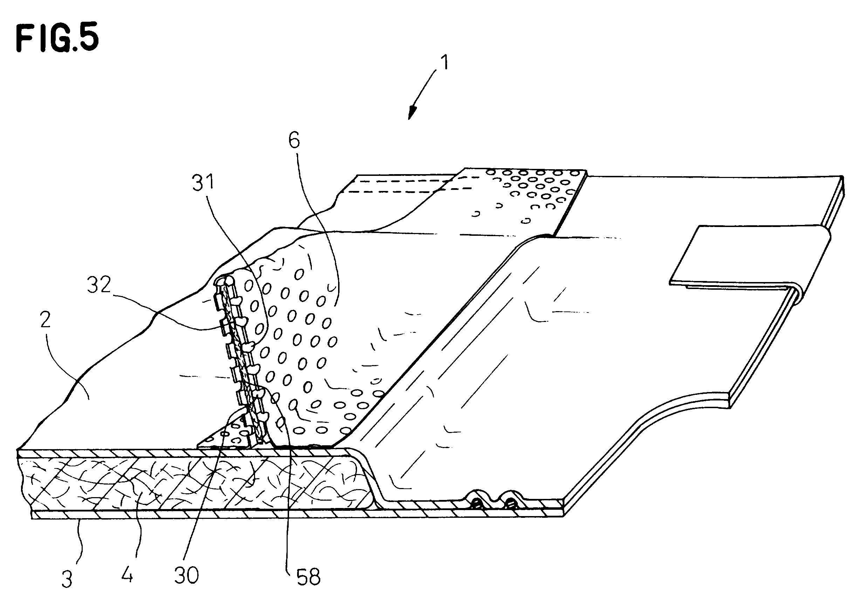

Fig. 5 is a view similar to Fig. 2 illustrating an alternative embodiment of the invention. In the diaper 1 according to this embodiment, each barrier cuff 6 is located above the core 4 adjacent the associated one of its transversely opposite side edges and contains therein a liquid-absorbent layer 58 made of a liquid-absorbent material such as a hydrophilic nonwoven fabric or tissue paper. The outlet openings 32 of the respective tubular passages 30 are normally in contact with the liquid-absorbent layer 58 which can serve, in turn, to absorb and contain therein the quantity of body fluids moving through the tubular passages 30.

To implement the diaper 1 of this invention, the top-and backsheets 2, 3 and the liquid-absorbent core 4 may be made of the respective materials conventionally employed for these components in the art. Both bonding between the different members and bonding between the mutually opposed portions of the same film 25 may be achieved either by any suitable means such as hot melt adhesive or by heating.

The absorbent article of the invention advantageously provides a good breathability between in- and exterior of the article during its actual use, since each barrier cuff is formed by folding the plastic film having the tubular passages offering a desired breathability in two with the outlet openings of the respective tubular passages being placed inside. The outlet openings of the tubular passages arranged in the respective sections of the plastic film thus folded in two are mutually opposed but in discontinuous relationship within the barrier cuff, so it is reliably avoided that a quantity of body fluids flowing into the barrier cuff through the tubular passages distributed on the one section might back flow through the tubular passages distributed on the other section of the folded film to the outside of the article.

With the embodiment in which the tubular passages have their inner diameters tapered inwardly of the barrier cuff, a quantity of body fluids can easily move inwardly but can not easily move outwardly of the barrier cuff, so a leakageproofing effect of the barrier cuff is correspondingly improved.

With the alternative embodiment in which the barrier cuff contains therein the liquid-absorbent layer and the outlet openings of the respective tubular passages are normally in contact with this liquid-absorbent layer, a quantity of body fluids having reached the tubular passages and adjacent thereto is positively absorbed by the liquid-absorbent layer and thereby an uncomfortable wet feeling otherwise occurring adjacent the barrier cuff can be effectively alleviated.

## Claims

1. A disposable absorbent article comprising a laminate structure composed of a liquid-permeable topsheet, a liquid-impermeable backsheet and a liquid-absorbent core disposed between these two sheets, and a pair of barrier cuffs made of plastic films and provided on transversely opposite sides of said laminate structure, wherein:
each of said barrier cuffs is configured to have a pair of mutually opposed walls with a space defined therebetween and a plurality of tubular passages extending from their inlet openings defined on an outer surface of said wall to their outlet openings defined on an inner surface of said wall.

2. A disposable absorbent article according to Claim 1, wherein each of said barrier cuffs comprises a film folded in two.

3. A disposable absorbent article according to Claim 1, wherein each of said barrier cuffs comprises a pair of separate films put one upon another.

4. A disposable absorbent article according to Claim 1 wherein each of said barriers comprises a liquid-absorbent material disposed within said space.

5. A disposable absorbent article according to Claim 1, wherein each of said tubular passages has its inner diameter tapered toward its outlet opening.

6. A disposable absorbent article according to Claim 1, wherein said barrier cuffs are provided at least on their outer surfaces with a hydrophilicity.

7. A disposable absorbent article according to Claim 1, wherein the outlet openings of said tubular passages defined on one of said mutually opposed walls are out of alignment with the outlet openings of said tubular passages defined on the other wall.

8. A disposable absorbent article according to Claim 1, wherein each of said barrier cuffs has a proximal edge and a distal edge, said distal edge being provided with an elastic member contractible in the longitudinal direction of said barrier cuff.
